(19) Europäisches Patentamt

European Patent Office

Office européen des brevets

(11) **EP 0 636 041 B1**

(12) **EUROPEAN PATENT SPECIFICATION**

(45) Date of publication and mention
of the grant of the patent:
**10.03.1999 Bulletin 1999/10**

(21) Application number: **93908749.0**

(22) Date of filing: **07.04.1993**

(51) Int. Cl.$^6$: **A61N 1/39**

(86) International application number:
**PCT/US93/03242**

(87) International publication number:
**WO 93/20892 (28.10.1993 Gazette 1993/26)**

(54) **SHORT-PULSE CARDIOVERSION**

HERZRHYTHMUSKORREKTUR MITTELS KURZER IMPULSE

CARDIOVERSION A IMPULSION COURTE

(84) Designated Contracting States:
**DE FR GB IT NL**

(30) Priority: **09.04.1992 US 866460**

(43) Date of publication of application:
**01.02.1995 Bulletin 1995/05**

(73) Proprietor: **ANGEION CORPORATION
Brooklyn Park, MN 55428-1088 (US)**

(72) Inventor: **KROLL, Mark, W.
Simi Valley, CA 93065 (US)**

(74) Representative:
**Schütz, Peter, Dipl.-Ing. et al
Dr. Dieter von Bezold
Dipl.-Ing. Peter Schütz
Dipl.-Ing. Wolfgang Heusler
Brienner Strasse 52
80333 München (DE)**

(56) References cited:
**EP-A- 0 326 290          GB-A- 2 190 296**

• **IEEE TRANSACTIONS ON BIOMEDICAL
ENGINEERING vol. 27, no. 1, January 1980,
pages 37 - 44 J. C. SCHUDER 'Transthoracic
Ventricular Defibrillation in the 100kg Calf with
Untruncated and Truncated Exponential Stimuli'**

## Description

The invention is concerned with an apparatus for producing a cardoversion waveform of less than about 5 joule to be delivered to two or more implanted electrodes in a human patient, as recited in the preamble of claim 1.

### Description of the Background Art

Implantable cardioverter-defibrillator (ICD) systems now on the market and in use for clinical studies typically employ capacitors of 120 to 180 microfarads to deliver a defibrillation countershock. In order to minimize the complexity of the ICD system, current ICDs use the same capacitor to deliver both the defibrillation countershock and a cardioversion pulse. For similar reasons, the same pulse duration for defibrillation countershocks is also used for cardioversion pulses. Because the energy employed for cardioversion pulses is some four to twenty times less than for defibrillation countershocks (1-5 joules versus 20-40 joules), the voltages used to deliver cardioversion pulses from the same capacitor are correspondingly smaller than the voltages for defibrillation countershocks. Unfortunately, low voltages in the later portions of a stimulation pulse are less effective in terminating tachycardia, and may even induce acceleration or fibrillation.

It is worthwhile to provide some background on ICDs. Defibrillation, or causing the cessation of chaotic and uncoordinated contraction of the ventricular myocardium by application of an electrical direct current and voltage, in its most primitive form goes back to the last century. [J. L. Provost and F Battle, "Sur Quelques Effets Des Decharges Electriques sir I Couer Des Mammifers, *"Comptes Rendus Hebdomadaires Des Seances t L "Acadmie Des Sciences*, Vol. 129, p. 1267, 1899.] Because of the large currents required for defibrillation, large-area electrodes are usually employed. [A. C. Gotten and J. Satterfield, "Factors Concerned In Defibrillation of the Heart, Particularly through the Unopened Chest," *Am. J. of Physiology,* Vol. 167, p. 81, 1951.]

For reasons of simplicity and compactness, capacitor-discharge systems are almost universally used in defibrillation. The discharge of a capacitor C through a resistance R results in a curve of voltage versus time, and hence, of current versus time as well, that is a declining exponential function, with a characteristic time constant given by the product RC. It has also been recognized for some time that the long - duration, low-amplitude "tail" of such a capacitor discharge pulse can be detrimental. [J. C. Skidder, G. A. Rahmoeller, and H. Stickily, "Transthoracic Ventricular Defibrillation with Triangular and Trapezoidal Waveforms," Circ. Res., Vol 19, p. 689, October 1966; W. A. Tacker, et al., "Optimum Current Duration for Capacitor-discharge Defibrillation of Canine Ventricles, " *J. Applied Physiology,* Vol 27, p. 480, October, 1969.] Although the exact reason for this detrimental effect is not known, plausible speculations exist, with one possibility being that field heterogeneities ease arrhythmias as significantly large regions of the heart. [P.S Chen, et al., "The Potential Gradient Field Created by Epicardial Defibrillation Electrodes in Dogs", *Circulation, Vol.* 74, p. 626, September 1986.]

A convenient way to eliminate the low-amplitude "tail" of a capacitor discharge is by switching, which is to say, simply opening the capacitor-load circuit after a predetermined time, or else when voltage has fallen to a particular value. For this reason, the time-truncated capacitor discharge has been extensively used after its effectiveness was first demonstrated. [J. C. Skidder, et al., "Transthoracic Ventricular Defibrillation in the Dog with Truncated and Untruncated Exponential Stimuli", *IEEE Trans. Biom. Eng., Vol*. BME-18, p. 410, November 1971.]

Two methods for specifying a time-truncated capacitor-discharge pulse in defibrillation systems have been extensively used. Unfortunately, neither method has involved systematic optimization of the pulse. Some manufacturers, such as Medtronic, Inc. in their PCD[®] product, simply specify pulse duration, although the physician can choose and adjust the value. A typical value might be a programmable duration being at least 7 ms. Other manufacturers, such as Cardiac Pacemakers, Inc. in their Ventak[®] product, specify the relative amount of voltage decline at the time of truncation, with a typical value of the decline being 65% of the initial voltage. It has become customary to use the term "tilt" to describe the relative amount of such voltage decline, expressed either as a decimal fraction or a percentage. In algebraic language,

$$tilt = (V_{initial} - V_{final})/V_{initial} \qquad\qquad Eq. 1$$

As a specific illustration of prior-art practice, one can review an existing ICD system that uses a 140-microfarad capacitor for defibrillation. When used with larger electrodes that typically yield a cardiac electrical resistance of 50 ohms, the system displays an RC time constant of 7 milliseconds. Using the specification of 65% tilt, one obtains a pulse duration of approximately 7 milliseconds, the RC time. Because all current ICD devices specify the same tilt or pulse duration for cardioversion pulses as for defibrillation countershocks, it necessarily follows that, if the initial voltage of a cardioversion pulse is only about 150 volts,compared to 600-750 volts for a defibrillation countershock, then the final voltage of the cardioversion pulse at the time of truncation can be as low as 40 to 50 volts, depending upon the initial cardioversion energy chosen. In particular, for a 1-joule pulse from the Ventak[®] system, for example, the trailing final voltage is 43 volts. Clinical experience with cardioversion pulses having durations and final voltages in these vicinities have shown an effectiveness of only 50 to 80 percent.

EP 0 326 290 A1 discloses an apparatus for applying an asymmetric biphasic exponential waveform countershock to the heart, useful in an implantable cardioverter or defibrillator. That apparatus comprises a high voltage charging circuit for charging a capacitor, and switch means for selectively controlling a time-truncated discharge of the capacitor through two electrodes to produce a therapeutical waveform comprising an electrical pulse of predetermined duration. The expontential decay of the capacitor discharge has a duration of 2 to 8 ms and should be equal to 0.7 times the RC-time constant provided by the capacitor and the resistance of the tissue between the electrodes.

The object of this invention is to provide for an appartus capable of delivering to implanted electrodes, pulses of considerably different energies depending on different requirements for cardioversion or defibrillation therapies.

This object is solved by an apparatus as defined in claim 1 and claim 4. Particular embodiments of the invention are defined by the features of the subclaims.

## SUMMARY OF THE INVENTION

In accordance with an embodiment of the invention, and apparatus for producing a cardioversion waveform of less than about 5 Joules to be delivered to two or more implanted electrodes in a human patient in response to a sensing of a myocardial arrhythmia in the patient comprises: capacitor means for storing a preselected amount of electrical energy for the cardioversion waveform; high voltage charging circuit means for charging the capacitor means to the preselected amount of electrical energy; and switch means for selectively controlling a time-truncated discharge of the capacitor means through the two or more electrodes in response to the sensing of the myocardial arrhythmia to produce the cardioversion waveform comprising at least one electrical pulse and having a predetermined optimum pulse duration between 0.5 and 3.0 ms set automatically by the apparatus based on an optimization of an effective current of the electrical pulse for the preselected amount of electrical energy stored in the capacitor means.

The apparatus is designed as an implantable device connected to at least two implanted electrodes located in a human patient to treat a myocardial arrhythmia, and operates by: a) sensing a myocardial, arrhythmia in the human patient; and (b) in response to the myocardial, arrhythmia, delivering from electrical energy stored in a capacitor system in the implantable device to the implanted electrodes a time-truncated cardioversion waveform of less than about 5 Joules comprising at least one electrical pulse and having a predetermined optimum pulse duration of preferably between 0.8 and 1.2 ms set automatically by the device based on an optimization of an effective current of the electrical pulse for a preselected amount of electrical energy stored in the capacitor system.

In particular, the inventive implantable cardioverter defibrillator for producing a cardioversion waveform of less than about 5 Joules and a defibrillation waveform of greater than about 5 Joules, both of which are to be delivered to at least two or more implanted electrodes in a human patient in response to a sensing of a myocardial arrhythmia in the patient comprises: capacitor means for storing a first preselected amount of electrical energy for the cardioversion waveform and a second preselected amount of electrical energy for the defibrillation waveform; high voltage charging circuit means for selectively charging the capacitor means to either the first or second preselected amounts of electrical energy; and switch means for selectively controlling a time-truncated discharge of the capacitor means through the two or more electrodes in response to the sensing of the myocardial arrhythmia to produce either the cardioversion waveform comprising at least one electrical pulse and having a first predetermined pulse duration, or the defibrillation waveform comprising at least one electrical pulse and having a second predetermined pulse duration, where the first predetermined pulse duration of the cardioversion waveform is less than the second predetermined pulse duration of the defibrillation waveform.

## BRIEF DESCRIPTION OF THE DRAWINGS

Figure 1 illustrates a preferred-embodiment monophasic pulse for cardioversion using the present invention;
Figure 2 illustrates the same preferred-embodiment monophasic pulse of the present invention, but with different specification;
Figure 3 illustrates a simplified representation of a circuit for generating the monophasic pulse of Figures 1 and 2;
Figure 4 illustrates an example of an embodiment for a biphasic pulse for cardioversion in accordance with the present invention;
Figure 5 constitutes a simplified representation of a circuit for generating the biphasic pulse of Figure 4; and,
Figure 6 illustrates defibrillation and cardioversion waveforms of the prior art and a cardioversion waveform for one embodiment of the present invention.

## DETAILED DESCRIPTION OF THE PREFERRED EMBODIMENT

The following detailed description, when considered in connection with the accompanying drawings in which like reference numerals designate like parts throughout the figures, describes some of the embodiments of the present invention.

In essence, the present invention allowes a cardioversion therapy that has a higher success rate and a lower complication rate than existing cardioversion therapies because the pulse duration of the cardioversion waveform is shorter than existing pulse durations of cardioversion and defibrillation countershocks, i.e., shorter than about 6 milliseconds. The pulse duration is optimized for an amount of electrical energy required by the high voltage charging circuit means from a battery system that is necessary to store the preselected amount of electrical energy in the capacitor means such that an overall displacement volume of the battery system is reduced and that the predetermined pulse duration of the cardioversion waveform essentially matches an average cardioversion chronaxie value of the myocardium of the human patient, whereby an overall pain level of the cardioversion waveform is reduced.

In addition to the hazard of supplying a low voltage trailing voltage to the heart, the longer cardioversion pulse duration supplied by existing ICDs constitutes a waste of energy. In tachycardia, heart cells that must be reset are in a resting state of polarization (Phase 4), a task requiring less energy than resetting cells that are in an actived state of depolarization (Phases 0-3), as in the case of ventricular fibrillation. It is believed that this phenomenon is responsible for the lower energies typically required by, and hence chosen for, cardioversion. The present invention takes this one step further by providing for a reduction of the pulse duration without sacrificing effectiveness, and eliminating the dangerous low-voltage tail from the cardioversion pulse.

One of the principal factors which has been essentially ignored in existing cardioversion therapies is the natural stimulation time constant of the heart itself. A characteristic time associated with far-field stimulation using electrodes relatively remote from the heart is in the neighborhood of 1 millisecond. For purposes of comparison, for a far-field defibrillation countershock the characteristic stimulation time is between 2 to 4 milliseconds, whereas the characteristic stimulation time for a pacing pulse is on the order of 300 microseconds. It will be understood that the characteristic time is an average time period and can vary slightly between patients and ICD systems, depending upon the myocardial resistance and the electrode size, for example. The elucidation of this characteristic time employs the concept of "chronaxie", that requires some background explanation.

The foundation for defining such a characteristic time is a family of mathematical neurophysiological models for tissue stimulation going back to the turn of the century, with the first important such model having been developed by Weiss. [G. Weiss, "Sur l Possibilite t Randall Comparable entire Eux le Appareils Suivant a l'Excitation Electrique," *Arch. Ital. t Biol.,* Vol. 35, p. 413, 1901.] He employed the *ballistic-rheotome* technique for pulse generation, wherein a rifle shot of known velocity is used to cut two wires in sequence, their spacing being set and measured. Cutting the first wire eliminated a short from a dc source, causing current to flow through the tissue under test, and cutting the second wire opened the circuit, terminating the pulse applied. Converting the electrical data into charge delivered by the pulse, Weiss found that the charge Q needed for stimulation was linearly dependent on pulse duration, d. Specifically,

$$Q = k_1 + k_2 d \qquad\qquad \text{Eq. 2}$$

Subsequently and similarly, the physiologist L. Lapicque collected substantial amounts of data on the amount of current required for tissue stimulation, using constant-current pulses of various durations. [L. Lapicque, "Definition Experimentelle t l'excitability," *Proc. Soc. t Biol., Vol. 77, p. 280,* 1909.]

Lapicque established an empirical relationship between the current I and the pulse duration d, having the form:

$$I = K_1 + (K_2 / d) \qquad\qquad \text{Eq. 3}$$

Equation 3 of Lapique shows that the necessary current and the pulse duration are related by a simple hyperbola, shifted away from the origin by the amount of the constant term $K_1$. Hence the stimulating current required in a pulse of infinite duration is $K_1$, a current value Lapicque termed the *rheobase.* Shortening the pulse required progressively more current, and the pulse duration that required a doubling of current for excitation, or $2K_1$, he termed the *chronaxie, $d_c$.* Substituting $2K_1$ and $d_c$ into Eq. 3 in place of I and d, respectively, yields

$$d_c = K_2 / K_1 \qquad\qquad \text{Eq. 4}$$

Lapicque's model described cell stimulation, rather than defibrillation, but Bourland demonstrated that defibrillation thresholds in dogs and ponies followed the Lapicque model, provided *average* current is used in the exercise. [J. D. Bourland, W. Tacker, and L. A. Jades, "Strength-Duration Curves for Trapezoidal Waveforms of Various Tilts for Transchest Defibrillation in Animals," *Med. Instr,* Vol. 12, p. 38, 1978.] In a companion paper, the same workers showed that

average current, $I_{ave}$, is a useful and consistent measure of defibrillation effectiveness for time-truncated pulses of a given duration through a substantial range of durations, from 2 to 20 milliseconds; in other words, so long as the exponential "tail" is eliminated, pulse effectiveness is not very dependant upon waveform details. [J. D. Bourland, W. Tacker, and L. A. Jades, "Comparative Efficacy of Damped Sine Waves and Square Wave Current for Transchest Defibrillation in Animals," *Med. Instr.*, Vol. 12, p. 42, 1978.]

The defibrillation chronaxie for the heart is usually between 2 milliseconds and 4 milliseconds, as is borne out by a substantial fund of published data. For cardioversion, the chronaxie is approximately 1 millisecond. For purposes of the present invention, the phrase "pulse duration set automatically by the apparatus" differentiates from those existing ICD devices that can be programmed to a short pulse duration by the physician. The phrase "is less than the second predetermined pulse duration for the defibrillation waveform" differentiates the ICD of the present invention from the existing ICDs, all of which have a fixed cardioversion pulse duration equal to their defibrillation pulse duration.

The general purpose of the present invention is to provide for optimization of the pulse duration of a cardioversion waveform. In doing so, the present invention builds upon the models of Lapicque and Weiss, and the finding of Bourland. To do this, a "sufficiency ratio" is defined; i.e. the ratio of Bourland's ruling average current and the current needed for defibrillation according to the Lapicque model for a heart of a given $K_1$, rheobase current, and a given $K_2$, a charge. Algebraically,

$$\text{Sufficiency ratio} = I_{ave} / (K_1 + K_2/d) \qquad \text{Eq. 5}$$

It is simply the ratio of Bourland's available therapeutic current (or the average current $I_{ave}$) to the current required according to the Lapicque formulation. Hence for a ratio of unity, the waveform of average current $I_{ave}$ and duration d will be able to defibrillate a heart characterized by $K_1$ and $K_2$. To review briefly, recall that the current $K_1$ and the charge $K_2$ characterize the sensitivity of particular tissue to electrical stimulation. The smaller these quantities are, the more sensitive is the tissue in question to electrical stimulation. The quotient of these two quantities yields a characteristic time, the chronaxie, which does not by itself indicate sensitivity or insensitivity.

Multiplying Eq. 5 through by the rheobase current $K_1$ yields an expression that, of course, has dimensions of amperes; noting from Eq. 4 that $K_1 d_c = K_2$, makes it possible to eliminate the heart-characterizing quantities $K_1$ and $K_2$ from this expression:

$$[I_{ave} / (1 + K_2 / K_1 d)] = [I_{ave} / (1 + d_c/d)] \qquad \text{Eq. 6}$$

Thus, one has an expression in the two pulse characterizing quantities $I_{ave}$ and d, and in one heart-characterizing quantity, $d_c$, the chronaxie time. Note that for an infinite pulse duration, this current simply equals the average current $I_{ave}$, but for a pulse of finite duration, it will be smaller than $I_{ave}$. This current, therefore, measures the effectiveness of a particular waveform in defibrillating a particular heart. For this reason the inventors have named it the effective current or $I_e$ so that the defining equation is

$$I_e = I_{ave} / (1 + d_c/d) \qquad \text{Eq. 7}$$

Note further that $I_e$ would be the same as $I_{ave}$ if one had a zero value of chronaxie time, $d_c$. In this sense, Eq. 7 constitutes a correction from actual average current necessitated by the chronaxie phenomenon. The effective current $I_e$ can be expressed in several ways:

$$\begin{aligned} I_e &= [I_{ave}d / (d_c + d)] \\ &= [(\text{delivered charge})/(d_c + d)] \\ &= CV(\text{tilt ratio})/(d_c + d) \end{aligned} \qquad \text{Eq. 8}$$

Applying this formulation to an existing ICD system employing C = 140 microfarad, tilt = 0.65, $V_i$ = 125 volts, and an inter-electrode resistance of R = 50 ohms (for a pulse duration of about 7 milliseconds) yields an effective current of:

$$I_e = [(140 \text{ F}) (125 \text{ V}) (0.65) / (1 \text{ ms} + 7 \text{ ms})] = 1.42 \text{ A}. \qquad \text{Eq. 9}$$

It is convenient to illustrate the kind of information obtainable from this analysis by using the hypothetical (and easy to calculate) case of a rectangular current pulse employed for cardioversion. With a current I delivered to the (cardiac) load resistance R, we can write the energy E of the pulse in joules as:

$$E = I^2 Rd \qquad \text{Eq. 10}$$

so that

$$I = (E / Rd)^{0.5} \qquad \text{Eq. 11}$$

The object, now, is to maximize the effective current $I_e$, which is given by

$$I_e = I_{ave} / (1 + d_c/d) \qquad \text{Eq. 12}$$
$$= (E / Rd)^{0.5} / (1 + d_c/d)$$

or,

$$I_e = (E / R)^{0.5} [ (1/d)^{0.5} / (1 + d_c/d) ] \qquad \text{Eq. 13}$$
$$= (E / R)^{0.5} [ (d)^{0.5} / (d_c + d) ]$$

Differentiating this expression with respect to d and equating the results to zero yields:

$$d = d_c \qquad \text{Eq. 14}$$

Thus, for a truly rectangular pulse, the optimal duration is simply the chronaxie time. This result has intuitive appeal, because one is matching the pulse duration to the natural time constant of the cardiac system. This result is known in pacing. [J. A. Pearce, e a., "Myocardial Stimulation with Ultrashort Duration Pulses", *PACE*, Vol. 5, p. 52, Jan.-Feb. 1982], and has been derived for defibrillation as well. It was, in fact, noted by Lapique. Hence, our result using the concept of effective current is consistent with prior knowledge regarding the optimum duration for ideal and hypothetical rectangular pulses.

It is instructive to calculate the current in an ideal hypothetical 1-joule rectangular pulse having a duration of 1 millisecond for comparison with the effective current obtained above for an existing truncated capacitor-discharge cardioversion/defibrillation pulse having a duration of 7 milliseconds. From Eq. 10,

$$I = [ (1J) / (50 \text{ ohm}) (1 \text{ ms}) ]^{0.5} = 4.47 \text{ A.} \qquad \text{Eq. 15}$$

Thus the 1-joule pulse in this hypothetical example yields an effective current over three times greater than that of a 1-joule pulse having a duration of 7 milliseconds, illustrating the virtue of a short pulse in spite of the hypothetical nature of the waveform in the present example. It is important to understand, however, that the characteristics of an ideal hypothetical rectangular pulse are not directly applicable to the performance of a truncated capacitor-discharge ICD.

Given that a truncated capacitor-discharge pulse is used in real life, the next task is to optimize such a pulse for cardioversion. A capacitor C will be charged to a voltage $V_i$ and discharged into a cardiac load resistance R, with time truncation to yield a pulse duration d. The effective capacitance value of the capacitor has been chosen with an eye to high-energy defibrillation. This fixed capacitor will be charged to a fixed voltage, resulting in the storage of a fixed energy in the capacitor. Neglecting converter losses, this energy will equal the energy drawn from the battery. That is, because it is not practical to recover energy left in the capacitor after a truncated pulse is delivered, if the goal is to minimize the amount of energy required by the ICD, it is the energy stored in the capacitor initially (rather than the energy delivered) that is relevant in determining the optimum pulse duration of the cardioversion waveform in terms of how to budget battery energy among the pacing function, the cardioversion function with multiple shocks, and the defibrillation function, also with multiple shocks. In contrast, if the goal is to minimize the myocardial damage and, hence, the pain associated with a cardioversion waveform, then it is energy delivered (rather than the energy stored) that is relevant in determining the optimum pulse duration of the cardioversion waveform.

With respect to the first goal, the first challenge is to maximize the effective current $I_e$ for a given energy stored initially in the capacitor.

Because the waveform is a declining exponential function, and given that $RC = \tau$, the system time constant, tilt as a decimal fraction can be written as follows:

$$\text{tilt} = 1 - \exp(-d/\tau) \qquad \text{Eq. 16}$$

Combining this expression and Eq. 8 yields:

$$I_e = CV[1\text{-exp}\,(\text{-}d/\tau)]/(d_c + d) \qquad \text{Eq. 17}$$

It is clear the $I_e$ vanishes at both extremes of d, so the intermediate extremum must be a maximum, defining explicitly the optimum waveform that can be achieved by varying pulse duration with a particular average current. To determine this optimum pulse duration, set

$$\frac{dI_e}{dd} = CV_i\,\frac{(d + d_c)\frac{1}{\tau}e^{-d/\tau} - (1\text{-}e^{-d/\tau})}{(d + d_c)^2} = 0 \qquad \text{Eq. 18}$$

Hence,

$$\frac{d + d_c}{\tau}\,e^{-d/\tau} - 1 + e^{-d/\tau} = \left(\frac{d_c + d}{\tau} + 1\right)e^{-d/\tau} - 1 = 0 \qquad \text{Eq. 19}$$

Using the system time constant $\tau$ = RC for normalization yields

$$z = d\,/\,\tau \qquad \text{Eq. 20}$$

and

$$\alpha = d_c\,/\,\tau \qquad \text{Eq. 21}$$

Using these definitions,

$$0 = (z + \alpha + 1)\,[\exp\,(\text{-}z)] - 1 \qquad \text{Eq. 22}$$

Next multiply through by $-e^z$ to obtain the simplified equation whose root is:

$$0 = [\exp\,(\text{-}z)] - z - \alpha - 1 \qquad \text{Eq. 23}$$

Because the equation is transcendental, it cannot be solved in dosed form, so define the function on the left-hand side as f(z) and the first approximation for its root as $z_0$. The Newton-Raphson method gives an approximate value for the root as

$$z' = z_0 - f(z_0)\,/\,f'(z_0) \qquad \text{Eq. 24}$$

Experience shows that waveforms with a tilt of about 65% are effective, and this corresponds to d = $\tau$ or $z_0$ = 1. Hence an appropriate approximate root is

$$\begin{aligned} z' &= 1 - (e - 1 - \alpha - 1)\,/\,(e\text{-}1) \\ &= (1 + \alpha)\,/\,(e - 1) \end{aligned} \qquad \text{Eq. 25}$$

Denormalization yields:

$$d = (\tau + d_c)\,/\,(e - 1) \qquad \text{Eq. 26}$$

for the approximate optimum value of pulse duration d as a function of chronaxie $d_c$ and system time constant.
Carrying through the optimization numerically shows that this estimate is valid within 0.2% for typical values of R, C, and $d_c$. Even for extreme values of these system and heart parameters, the approximate value of optimum duration produces a value for the current Ie that is within 2% of the optimum. Since (e - 1) = 1.72, the optimum pulse duration is approximately (and somewhat larger than) the average of the system's time constant and the heart's characteristic time $d_c$. In other words, the optimum pulse duration is a compromise between the two characteristic times involved.
To illustrate the application of this analysis, let us return to the case of an existing ICD system for producing a cardioversion waveform employing C = 140 microfarads, $V_i$ = 125 volts, and an inter-electrode resistance of R = 50 ohms. We now determine the optimal pulse duration d for this case, describing a preferred embodiment of the present invention in the process. From Eq. 26, recalling that $d_c$ = 1 millisecond for cardioversion,

$$d = [ (7 \text{ ms} + 1 \text{ ms})/(e\text{-}1) ] = 4.65\text{ms} \qquad \text{Eq. 27}$$

For this pulse duration, the tilt amounts to 49%, or 0.49. Hence the effective current from Eq. 8, once more, is

$$I_e = [(140 \text{ F}) (125 \text{ V}) (0.49) / (1 \text{ ms} + 4.65 \text{ ms})] = 1.52 \text{ A.} \qquad \text{Eq. 28}$$

This constitutes an increase over the prior-art value (1.42 ampere) for the same system in spite of the fact that less energy is delivered by the truncated cardioversion pulse with its optimal shorter duration. The optimal pulse duration brings the further benefit of eliminating the lingering low voltage portions of the pulse that can lead to ventricular fibrillation. The optimized pulse as described in this embodiment has a final voltage of $V_f$ = 64 V , while for the 7 millisecond cardioversion pulse of an existing ICD, the value is $V_f$ = 44 V . The further advantage is that the 64-V value exists at a time less then 5 ms, while the 44-V value of the prior art exists at 7 ms. The delivery of an optimized pulse duration such as this for cardioversion is one of the essences of the present invention.

In addition to the advantages just cited, there are further benefits to be gained by maximizing the quotient of effective current by pulse energy. Diminished energy reduces the inevitable damage to myocardial tissue. Even further, it diminishes pain. Thus, an impressive set of betterments flow from the short cardioversion pulse of the present invention.

The discussion thus far has addressed the issue of optimization for the case where energy initially stored in the battery is held constant. There is, however, a different problem that is worth addressing. That is, the case where *delivered* energy is held constant. This implies a higher initial voltage $V_i$ as pulse duration shrinks. But, this further involves a diminished tilt, and hence, a more nearly rectangular pulse. Conceding that increased amounts of energy initially must be delivered from the battery to the capacitor for each such pulse, and that any energy remaining in the capacitor cannot be recovered, and hence is wasted, we nonetheless believe this line of inquiry to be worthwhile.

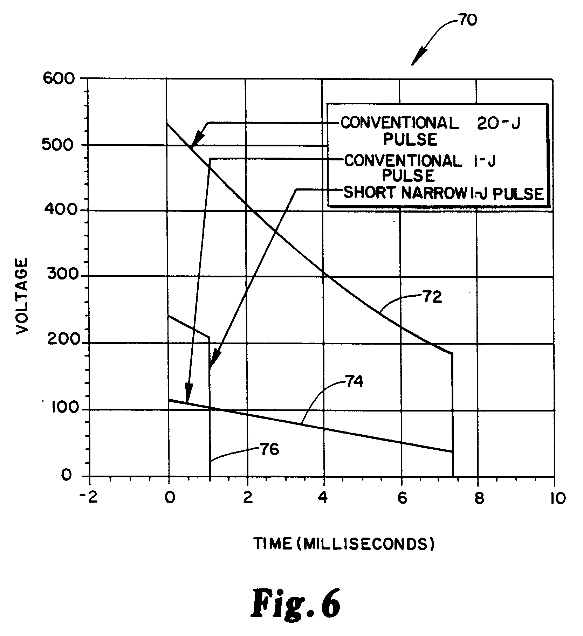

Note first that going from a 7-ms conventional pulse to a 1-ms pulse reduces tilt from about 65% to about 14%, as is illustrated in Figure 6. Because this is an excellent approximation to a rectangular form, we receive the benefit of the kind of pulse connected with the calculation in Eq. 15. Further, as demonstrated in Eq. 14, the optimal duration of a rectangular pulse is simply the chronaxie, $d_c$. Thus, the insights involving the short pulse of Figure 6 yields an optimal duration only slightly larger than the chronaxie, and having an effective current within 1% of that obtained by simply using the chronaxie as the optimum pulse duration.

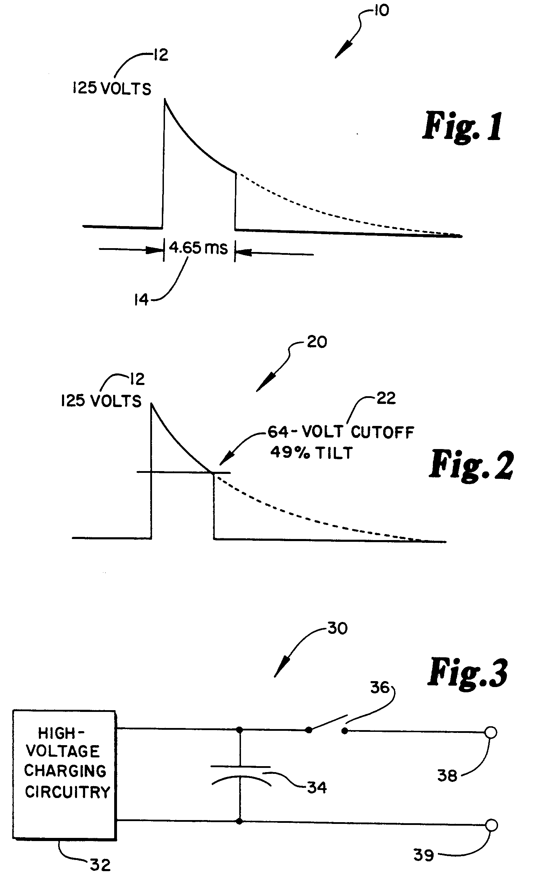

Cardioversion can be accomplished by using a monophasic wave, such as that illustrated in Figures 1 and 2. The first shows a preferred embodiment of the present invention, with the pulse properties specified in terms of initial voltage and pulse duration; the second employs initial voltage and tilt, producing the same result in this case. Figure 1 illustrates the waveform 10 of a preferred embodiment monophasic pulse for cardioversion of the present invention, specified by means of an initial voltage 12 and a duration 14. Figure 2 illustrates the waveform 20 of a preferred embodiment monophasic pulse for cardioversion of the present invention, specified by means of an initial voltage 12 and a tilt 22.

A simplified representation of a circuit for generating such a monophasic pulse is illustrated in Figure 3. A representation 30 of an electrical circuit for generating the monophasic pulse of Figures 1 and 2, including high-voltage charging circuitry 32 for the capacitor 34, and a switch 36 that is closed to deliver the pulse to the cardiac electrodes 38 and 39.

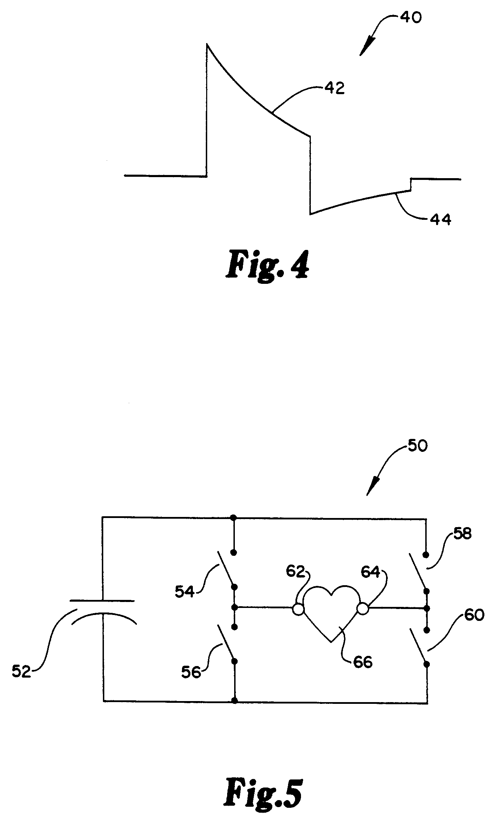

Cardioversion, like defibrillation, can be accomplished by means of a multiphasic pulse as well as a monophasic pulse. An example of a multiphasic pulse is the biphasic pulse illustrated in Figure 4, and the corresponding circuit is shown in Figure 5. In the biphasic case, the present method is used to fix the duration of the first phase, and then one usually lets the duration of the second phase equal a fraction of that determined to be optimal for the first.

Figure 4 illustrates the waveform 40 of an example of a biphasic pulse for cardioversion of the present invention, including a first phase 42 and a contiguous second phase 44. Figure 5 illustrates a representation 50 of an electrical circuit for generating the biphasic waveform 40 of Figure 4, including a capacitor 52, the four switches 54, 56, 58, and 60 that are able to reverse polarity in going from the first phase 42 of Figure 4 to the second phase 44 of Figure 4 for purposes of delivering the biphasic waveform 40 of Figure 4 to the cardiac electrodes 62 and 64 positioned on or near the heart 66.

Figure 6 illustrates a set 70 of voltage-versus-time waveforms, including the defibrillation waveform 72 of the prior art, the cardioversion waveform 74 of the prior art, and the cardioversion waveform 76 for one preferred embodiment of the present invention.

The model that is developed in the present invention is based upon the pioneering neurophysiological models of Lapicque and Weiss. The present model determines mathematically the optimum pulse duration, d, for a truncated capacitor-discharge waveform employed for cardioversion. It comprehends the system time constant, RC, where R is tissue resistance and C is the value of the capacitor being discharged, and also the heart's cardioversion chronaxie, $d_c$ defined by Lapicque, which is a characteristic time associated with the stimulation of heart tissue.

The present model and analysis find the optimum pulse duration for the case of fixed energy stored to be roughly the average of RC and $d_c$, appreciably smaller than the value employed in the prior art. The optimized-pulse prescription of this invention can be applied to a monophasic waveform, or to the first phase of a biphasic waveform. In the latter case, when it is applied to the first phase, the second phase can be specified to have equal or lesser duration than the first.

When energy delivered to the heart is taken to be constant, and the waveform is optimized on that basis, then the pulse duration approximates the chronaxie of the myocardial tissue.

## Claims

1. Apparatus for producing a cardioversion waveform of less than about 5 joules to be delivered to two or more implanted electrodes in a human patient, comprising:

    - capacitor means (34) for storing the preselected amount of the electrical energy for the cardioversion waveforms;
    - a high voltage charging circuit (32) for charging the capacitor means to the preselected amount of the electrical energy;
    - and switch means (36) for selectively controlling a time-truncated discharge of the capacitor means through said electrodes in response to the sensing of the myocardial arrythmia in the patient to produce the cardioversion wave-form comprising at least one electrical pulse,
    **characterized in that**
    - the pulse duration is arranged to be between 0.5 and 3.0 milliseconds, preferably between 0.8 and 1.2 milliseconds,
    - and in that said apparatus is designated to also produce a defibrillation waveform of more than about 5 joules and comprising at least one electrical pulse having a longer duration than said cardioversion pulse.

2. Apparatus as in claim 1, **characterized in that** the pulse duration is measured by a percentage tilt decay between an initial voltage value and a final voltage value where the percentage tilt is between 5 percent and 20 percent, preferably between 12 percent and 16 percent.

3. Apparatus as claimed in any proceeding claim, **characterized in that** the pulse duration is determined by the switch means (36) to be the sum of: a first value derived from a first predetermined percentage of an RC time constant, with R being a myocardial tissue resistance value and C being the effective capacitance value of the means; and a second value derived from a second predetermined percentage of a cardioversion chronaxie value.

4. An implantable cardioverter for producing the cardioversion waveform of less than 5 joules to be delivered to at least two or more implanted electrodes in a human patient in response to a sensing of a myocardial arrythmia in the patient, comprising: capacitor means (34) for storing a preselected amount of electrical energy for the cardioversion; high voltage charging circuit means (32) for charging said capacitor means to a preselected amount of electrical energy; and switch means (36) for controlling truncated discharge of the capacitor means through the two or more electrodes in response to the sensing of the myocardial arrythmia, characterized by being also operable as a defibrillator for producing a defibrillation waveform of greater than about 5 joules, whereby said high voltage charging circuit means (32) and said capacitor means (34) are selected to also respectively charge and store a preselected amount of electrical energy for the defibrillation waveform; and said switch means (36) selectively controlling discharge of said capacitor means (34) to produce either the cardioversion waveform or the defibrillation waveform comprising at least one electrical pulse and having a first and a second predetermined waveform duration, respectively, where the first predetermined waveform duration of the cardioversion waveform is less than the second predetermined waveform duration of the defibrillation waveform.

## Patentansprüche

1. Vorrichtung zur Erzeugung einer Kardioversions-Wellenform von weniger als etwa 5 Joule, die an zwei oder mehr einem menschlichen Patienten implantierte Elektroden zu übertragen ist, mit

- einer Kapazität (34) zur Speicherung des vorgewählten Betrages elektrischer Energie für die Kardioversions-Wellenformen;
- einer Hochspannungsladeschaltung (32) zur Aufladung der Kapazität auf den vorgewählten elektrischen Energiebetrag;
- einem Schalter (36) zur selektiven Steuerung einer zeitlich abgebrochenen Entladung der Kapazität durch die Elektroden in Abhängigkeit von der Feststellung der Myokard-Arrhythmie beim Patienten zur Erzeugung der Kardioversions-Wellenform mit mindestens einen elektrischen Impuls,
  **dadurch gekennzeichnet**, daß
- die Impulsdauer zwischen 0,5 und 3,0 Millisekunden, vorzugsweise zwischen 0,8 und 1,2 Millisekunden, bemessen ist,
- und daß die Vorrichtung so ausgelegt ist, daß sie eine Defibrillations-Wellenform von mehr als etwa 5 Joule erzeugt mit mindestens einem elektrischen Impuls längerer Dauer als der Kardioversionsimpuls.

2. Vorrichtung nach Anspruch 1, **dadurch gekennzeichnet**, daß die Impulsdauer über die prozentuale Neigung des Abfalls von einem Anfangsspannungswert auf einen Endspannungswert gemessen wird, wobei der prozentuale Abfall zwischen 5 und 20%, vorzugsweise zwischen 12 und 16% liegt.

3. Vorrichtung nach einem der vorstehenden Ansprüche, **dadurch gekennzeichnet**, daß die Impulsdauer mit Hilfe des Schalters (36) bestimmt wird als Summe von:

   einem ersten Wert, der aus einem ersten vorbestimmten Prozentsatz einer RC-Zeitkonstante abgeleitet ist, bei welcher R ein Myokardgewebe-Widerstandswert und C der effektive Wert der Kapazität ist,
   und einem zweiten Wert, der aus einem zweiten vorbestimmten Prozentsatz eines Kardioversions-Chronaxie-Wertes abgeleitet ist.

4. Implantierbarer Kardioverter zur Erzeugung der Kardioversions-Wellenform von weniger als 5 Joule, die an mindestens zwei oder mehr einem menschlichen Patienten implantierte Elektroden bei Feststellung einer Myokard-Arrhythmie beim Patienten zu übertragen sind, mit

   einer Kapazität (34) zur Speicherung eines vorgewählten Betrages elektrischer Energie für die Kardioversion;
   einer Hochspannungsladeschaltung (32) zur Aufladung der Kapazität auf einen vorbestimmten Betrag elektrischer Energie;
   und einem Schalter (36) zur Steuerung einer abgebrochenen Entladung der Kapazität durch die zwei oder mehr Elektroden bei Feststellung der Myokard-Arrhythmie, **dadurch gekennzeichnet**, daß er auch als Defibrillator betreibbar ist zur Erzeugung einer Defibrillations-Wellenform von mehr als etwa 5 Joule, wobei die Hochspannungsladeschaltung (32) und die Kapazität (34) so bemessen sind, daß ein vorgewählter Betrag elektrischer Energie für die Defibrillations-Wellenform aufgeladen und gespeichert wird, und daß der Schalter (36) die Entladung der Kapazität (34) selektiv so steuert, daß entweder die Kardioversions-Wellenform oder die Defibrillations-Wellenform mit mindestens einem elektrischen Impuls und einer ersten bzw. zweiten vorbestimmten Dauer der Wellenform erzeugt werden, wobei die erste vorbestimmte Dauer der Kardioversions-Wellenform kleiner als die zweite vorbestimmte Dauer der Defibrillations-Wellenform ist.

## Revendications

1. Appareil pour produire une forme d'onde de cardioversion inférieure à environ 5 joules devant être délivrée à deux électrodes ou plus implantées chez un patient humain, comprenant :

   - des moyens formant condensateur (34) pour stocker la valeur présélectionnée d'énergie électrique pour les formes d'onde de cardioversion ;
   - un circuit de charge à haute tension (32) pour charger les moyens formant condensateur à la valeur présélectionnée d'énergie électrique ;
   - des moyens de commutation (36) pour commander de manière sélective, une décharge tronquée dans le temps, des moyens formant condensateur par le biais desdites électrodes en réponse à la détection de l'arythmie myocardiale chez le patient afin de produire la forme d'onde de cardioversion comprenant au moins une impulsion électrique,
     caractérisé en ce que
   - la durée d'impulsion est conçue pour être comprise entre 0,5 et 3,0 millisecondes, de préférence entre 0,8 et 1,2 milliseconde,

- et en ce que ledit appareil est conçu pour produire également une forme d'onde de défibrillation de plus de 5 joules environ et comprenant au moins une impulsion électrique présentant une durée plus longue que ladite impulsion de cardioversion.

2. Appareil selon la revendication 1, caractérisé en ce que la durée d'impulsion est mesurée par une décroissance d'inclinaison en pourcentage entre une valeur de tension initiale et une valeur de tension finale où l'inclinaison en pourcentage est comprise entre 5 pour cent et 20 pour cent, de préférence entre 12 pour cent et 16 pour cent.

3. Appareil selon l'une quelconque des revendications précédentes, caractérisé en ce que la durée d'impulsion est déterminée par les moyens de commutation (36) pour être la somme de : une première valeur dérivée d'un premier pourcentage prédéterminé d'une constante de temps RC, R étant une valeur de résistance du tissu myocardial et C étant la valeur de capacitance effective des moyens ; et une seconde valeur dérivée d'un second pourcentage prédéterminé d'une valeur de chronaxie de cardioversion.

4. Défibrillateur implantable pour produire la forme d'onde de cardioversion inférieure à 5 joules devant être délivrée à au moins deux électrodes implantées ou plus chez un patient humain en réponse à une détection d'une arythmie myocardiale chez le patient, comprenant : des moyens formant condensateur (34) pour stocker une valeur présélectionnée d'énergie électrique pour la cardioversion ; des moyens formant circuit de charge à haute tension (32) pour charger lesdits moyens formant condensateur à une valeur présélectionnée d'énergie électrique ; et des moyens de commutation (36) pour commander une décharge tronquée des moyens formant condensateur par l'intermédiaire des deux électrodes ou plus en réponse à la détection de l'arythmie myocardiale, caractérisé en ce qu'il peut également fonctionner comme défibrillateur afin de produire une forme d'onde de défibrillation supérieure à 5 joules environ, lesdits moyens formant circuit de charge à haute tension (32) et lesdits moyens formant condensateur (34) étant choisis pour charger et stocker également de manière respective une valeur présélectionnée d'énergie électrique pour la forme d'onde de défibrillation ; et lesdits moyens de commutation (36) commandant de manière sélective une décharge desdits moyens formant condensateur (34) afin de produire soit la forme d'onde de cardioversion, soit la forme d'onde de défibrillation comprenant au moins une impulsion électrique et présentant respectivement une première et une seconde durée de forme d'onde prédéterminée, où la première durée de forme d'onde prédéterminée de la forme d'onde de cardioversion est inférieure à la seconde durée de forme d'onde prédéterminée de la forme d'onde de défibrillation.

*Fig. 1*

*Fig. 2*

*Fig. 3*

*Fig. 4*

*Fig.5*

Fig. 6

14